(19) Europäisches Patentamt
European Patent Office
Office européen des brevets

(11) Veröffentlichungsnummer: **0 138 002**
**A2**

# (12) EUROPÄISCHE PATENTANMELDUNG

(21) Anmeldenummer: **84110328.6**

(22) Anmeldetag: **30.08.84**

(51) Int. Cl.⁴: **A 01 N 43/40**

(30) Priorität: **07.09.83 DE 3332272**

(43) Veröffentlichungstag der Anmeldung: **24.04.85**
**Patentblatt 85/17**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Marzolph, Gerhard, Dr., Semmelweisstrasse 87a, D-5000 Köln 80 (DE)**
Erfinder: **Lunkenheimer, Winfried, Dr., Bismarckstrasse 29, D-5600 Wuppertal 1 (DE)**
Erfinder: **Fedtke, Carl, Dr., Wehrhelderstrasse 5, D-5000 Köln 80 (DE)**

## (54) Herbizide Mittel enthaltend Photosynthesehemmer-Herbizide in Kombination mit Pyridincarbonsäureamiden.

(57) Die neuen synergistischen Wirkstoffkombinationen bestehend aus (1) einem Photosynthesehemmer-Wirkstoff (Herbizid) und (2) einem Pyridincarbonsäureamid der allgemeinen Formel (II) (Synergist)

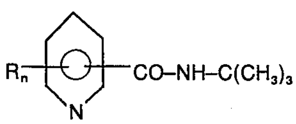

(II)

in welcher

R für Alkyl, Halogen, Nitro, Cyano, Alkoxy, gegebenenfalls substituiertes Phenyl oder gegebenenfalls substituiertes Benzyloxy steht und der Index

n für die Zahlen 0, 1, 2 oder 3 steht,

weisen eine besonders hohe herbizide Wirksamkeit auf.

Charakteristische Beispiele für die Photosynthesehemmer-Wirkstoffe sind Metribuzin, Ametridione, Methabenzthiazuron, Linuron, Lenacil, Atrazin sowie 4-Amino-6-tert.-butyl-3-ethylthio-1,2,4-triazin-5-on.

EP 0 138 002 A2

BAYER AKTIENGESELLSCHAFT 5090 Leverkusen, Bayerwerk

Konzernverwaltung RP
Patentabteilung Bi/li-c

III

# Herbizide Mittel enthaltend Photosynthesehemmer-Herbizide in Kombination mit Pyridincarbonsäureamiden

Die vorliegende Erfindung betrifft neue herbizide synergistische Wirkstoffkombinationen, die aus bekannten Photosynthesehemmer-Herbiziden einerseits und aus bestimmten, weitgehend bekannten Pyridincarbonsäureamiden andererseits bestehen.

Es ist bereits bekannt geworden, daß bestimmte Herbizide, wie z. B. 4-Amino-6-tert.-butyl-3-methylthio- bzw. -ethylthio-1,2,4-triazin-5-on; 1-Amino-3-(2,2-dimethylpropyl)-6-(ethylthio)-1,3,5-triazin-2,4-dion; 6-Chlor-2-ethylamino-4-isopropylamino-1,3,5-triazin; 1-Methoxy-1-methyl-3-(3,4-dichlorphenyl)-harnstoff; 1,3-Dimethyl-1-(benzo-1,3-thiazol-2-yl)-harnstoff oder 3-Cyclohexyl-5,6-trimethylenuracil, photosynthese-hemmende Eigenschaften besitzen (vergl. z. B. Carl Fedtke, Biochemistry and Physiology of Herbicide Action, Springer Verlag, 1982). Nachteilig bei diesen herbiziden Verbindungen ist

jedoch, daß nicht immer alle vorkommenden Unkräuter und Ungräser voll erfaßt werden oder aber, daß bei entsprechend hohen Aufwandmengen einige Kulturpflanzenarten teilweise geschädigt werden.

Es wurde gefunden, daß die neuen Wirkstoffkombinationen, bestehend aus

a) einem Photosynthesehemmer-Wirkstoff (Herbizid) und

b) einem Pyridincarbonsäureamid der allgemeinen Formel (II) (Synergist)

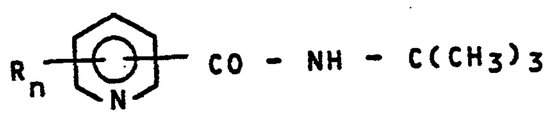

(II)

in welcher

R für Alkyl, Halogen, Nitro, Cyano, Alkoxy, gegebenenfalls substituiertes Phenyl oder gegebenenfalls substituiertes Benzyloxy steht und der Index

n für die Zahlen 0, 1, 2 oder 3 steht,

eine besonders hohe herbizide Wirksamkeit aufweisen.

Überraschenderweise ist die herbizide Wirksamkeit der erfindungsgemäßen Wirkstoffkombinationen wesentlich höher als die Summe der Wirkungen der einzelnen Wirkstoffe.

Insbesondere besitzen die weitgehend bekannten Pyridincarbonsäureamide der allgemeinen Formel (II) bei den üblichen Aufwandmengen keine eigene herbizide Wirkung, bewirken aber die Steigerung der herbiziden Wirkung der Photosynthesehemmer-Wirkstoffe. Somit ist der hier gefundene synergistische Effekt völlig unerwartet und überraschend.

Da der synergistische Effekt auch solche Unkräuter betrifft, die durch die verwendeten Photosynthesehemmer-Wirkstoffe bei alleiniger Ausbringung in üblichen Aufwandmengen nur unzureichend geschädigt oder gar nicht erfaßt werden, stellen die erfindungsgemäßen synergistischen Wirkstoffkombinationen eine wertvolle Bereicherung der Technik dar.

Als Photosynthesehemmer-Wirkstoffe für die erfindungsgemäßen Wirkstoffkombinationen seien vorzugsweise die folgenden der allgemeinen Formeln (I-A) bis (I-J) genannt:

(A) Triazinon-Derivate der Formel

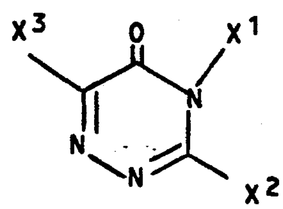

(I-A)

in welcher

$X^1$ für Amino, gegebenenfalls substituiertes Alkylidenamino oder Alkyl mit 1 bis 2 Kohlenstoffatomen steht;

$X^2$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^3$ für gegebenenfalls durch Halogen substituiertes tert.-Butyl oder gegebenenfalls substituiertes Phenyl steht;

(B) Triazindion-Derivate der Formel

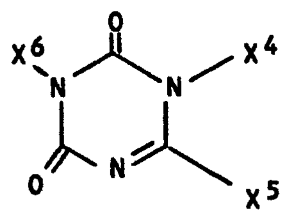

(I-B)

in welcher

$X^4$ für Amino, gegebenenfalls substituiertes Alkylidenamino oder Alkyl mit 1 bis 2 Kohlenstoffatomen steht;

$X^5$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^6$ für Alkyl mit 1 bis 6 Kohlenstoffatomen oder gegebenenfalls substituiertes Phenyl steht;

(C) Triazin-Derivate der Formel

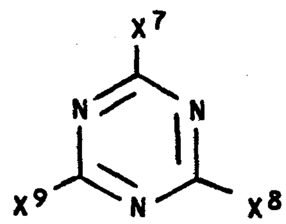

(I-C)

in welcher

$X^7$ für Chlor, Alkoxy oder Alkylthio mit jeweils 1 bis 2 Kohlenstoffatomen steht;

$X^8$ für Alkylamino mit 1 bis 4 Kohlenstoffatomen im Alkylteil steht; und

$X^9$ für gegebenenfalls durch Cyano substituiertes Alkyl mit 1 bis 4 Kohlenstoffatomen steht;

(D) Harnstoff-Derivate der Formel

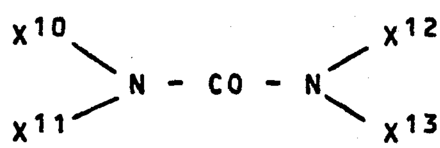

(I-D)

in welcher

$X^{10}$ für gegebenenfalls substituiertes Phenyl, Benzthiazolyl oder gegebenenfalls substituiertes Thiadiazolyl steht;

$X^{11}$ für Wasserstoff oder Methyl steht;

$X^{12}$ für Methyl steht; und

$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht;

(E) Carboxanilid-Derivate der Formel

$$X^{14} - CO - NH - X^{15} \qquad (I\text{-}E)$$

in welcher

$X^{14}$ für Alkyl mit bis zu 6 Kohlenstoffatomen, Alkoxy mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht;

(F) Uracil-Derivate der Formel

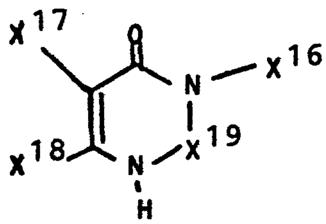

(I-F)

in welcher

$X^{16}$ für Alkyl mit 1 bis 6 Kohlenstoffatomen oder Cycloalkyl mit 5 bis 7 Kohlenstoffatomen steht;

$X^{17}$ für Halogen steht;

0138002

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die -CO-oder $-SO_2$-Gruppe steht;

(G) Biscarbamat-Derivate der Formel

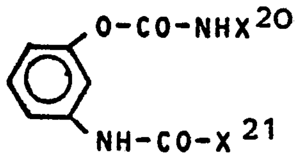

(I-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder Dialkylamino mit 1 bis 2 Kohlenstoffatomen in jedem Alkylteil steht;

(H) Pyridazinon-Derivate der Formel

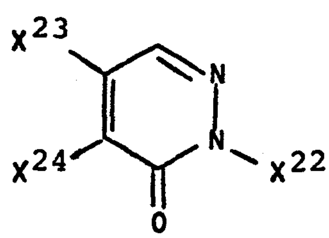

(I-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht;

(J) Hydroxybenzonitril-Derivate der Formel

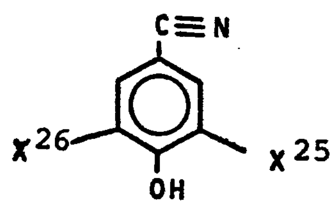

(I-J)

in welcher

$X^{25}$ für Halogen steht; und

$X^{26}$ für Halogen steht.

Besonders bevorzugt sind folgende Photosynthesehemmer-Wirkstoffe der allgemeinen Formeln (I-A) bis (I-J):

(A) Triazinon-Derivate der Formeln

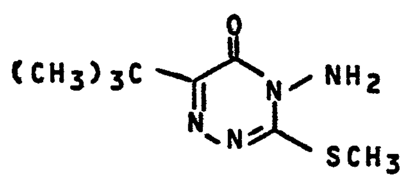

(I-A-1)
(Metribuzin)

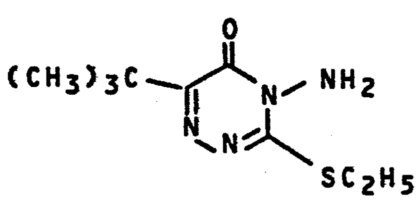

(I-A-2)

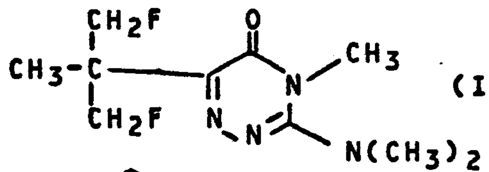

(I-A-3)

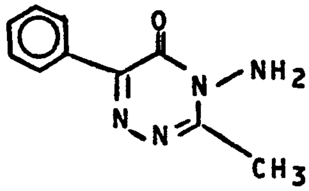

(I-A-4)
(Metamitron)

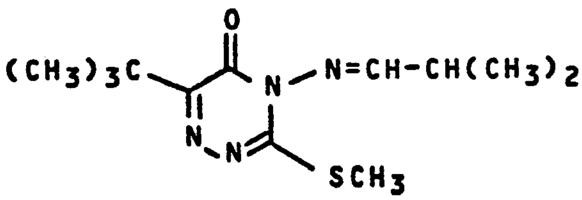

(I-A-5)
(Isomethiozin)

(B) Triazindion-Derivate der Formeln

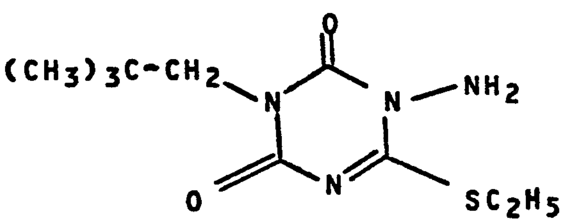

(I-B-1)
(Ametridione)

(C) Triazin-Derivate der Formeln

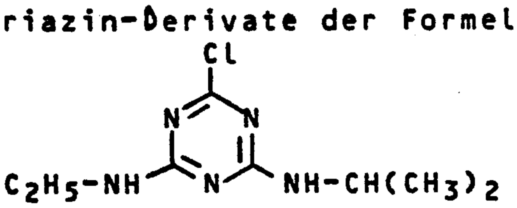

(I-C-1)
(Atrazine)

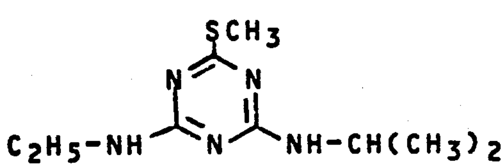

(I-C-2)
(Ametryne)

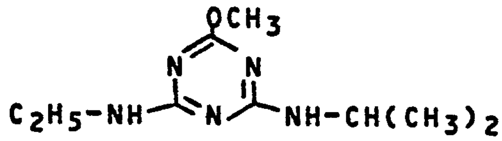

(I-C-3)
(Atraton)

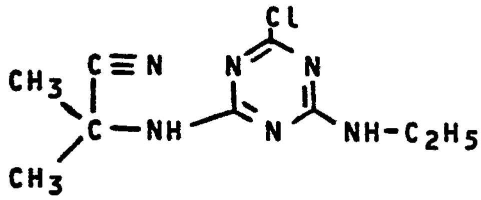

(I-C-4)
(Cyanazine)

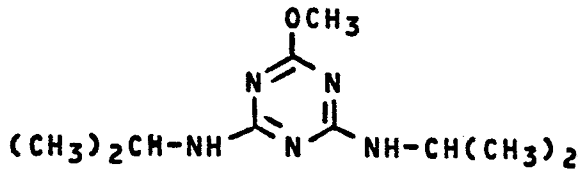

(I-C-5)
(Prometon)

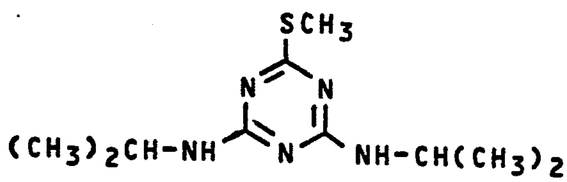

(I-C-6)
(Prometryne)

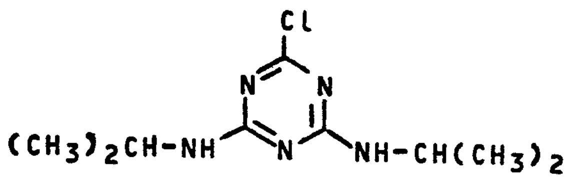

(I-C-7)
(Propazine)

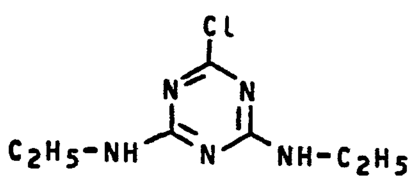

(I-C-8)
(Simazine)

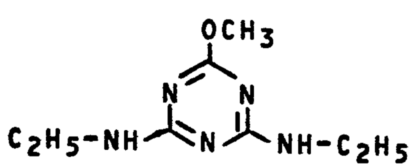

(I-C-9)
(Simeton)

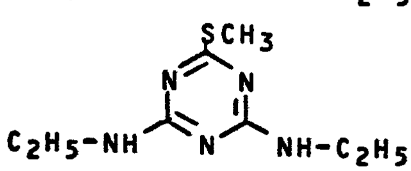

(I-C-10)
(Simetryne)

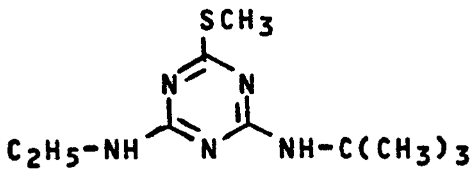

(I-C-11)
(Terbutryne)

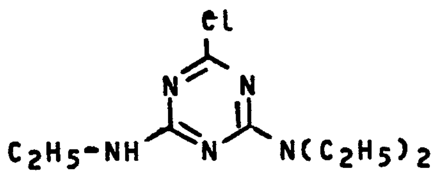

(I-C-12)
(Trietazine)

(D) Harnstoff-Derivate der Formeln

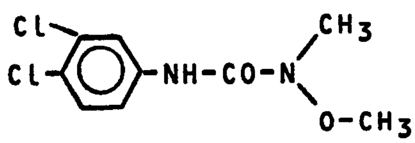

(I-D-1)
(Linuron)

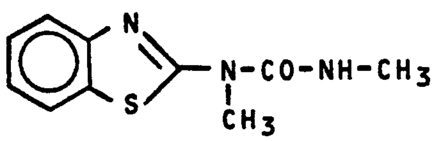

(I-D-2)
(Methabenzthiazuron)

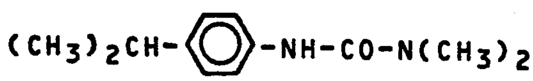

(I-D-3)
(Isoproturon)

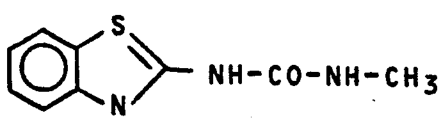

(I-D-4)
(Benzthiazuron)

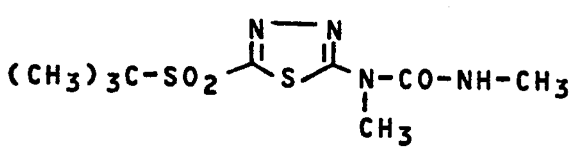

(I-D-5)
(Buthiuron)

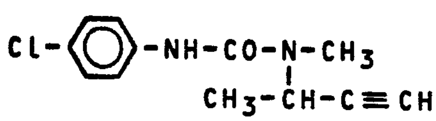

(I-D-6)
(Buturon)

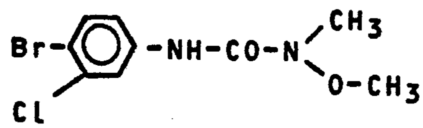

(I-D-7)
(Chlorbromuron)

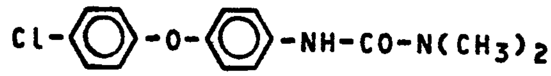

(I-D-8)
(Chloroxuron)

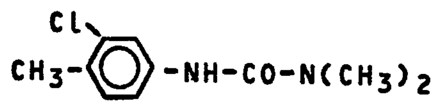

(I-D-9)
(Chlortoluron)

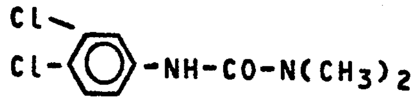

(I-D-10)
(Diuron)

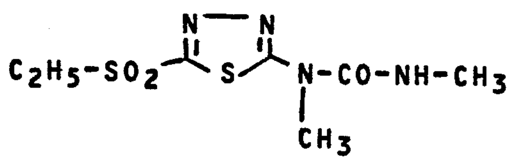

(I-D-11)
(Ethidimuron)

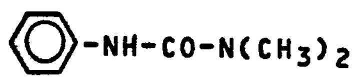

(I-D-12)
(Fenuron)

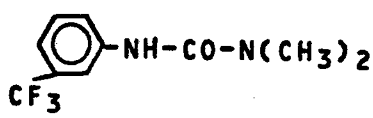

(I-D-13)
(Fluometuron)

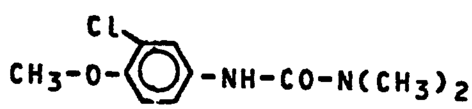

(I-D-14)
(Metoxuron)

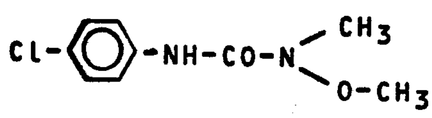

(I-D-15)
(Monolinuron)

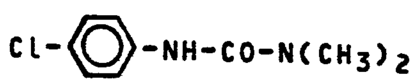

(I-D-16)
(Monuron)

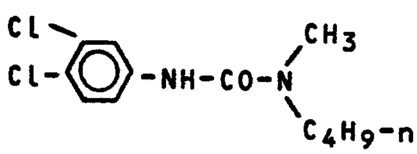

(I-D-17)
(Neburon)

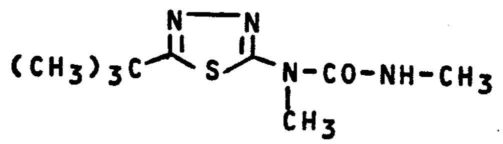

(I-D-18)
(Tebuthiuron)

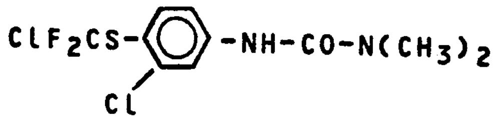

(I-D-19)
(Thiochlormethyl)

(E) Carboxanilid-Derivate der Formeln

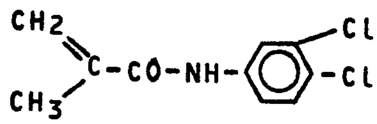

(I-E-1)
(Chlorancryl)

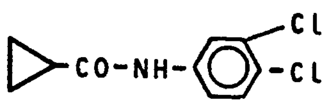

(I-E-2)
(Cypromid)

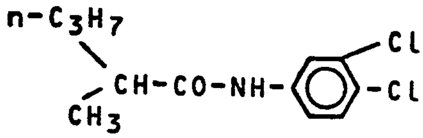

(I-E-3)
(Karsil)

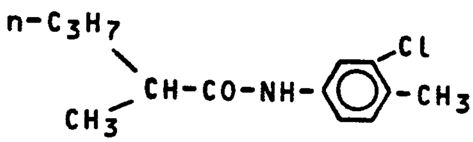

(I-E-4)
(Pentanochlor)

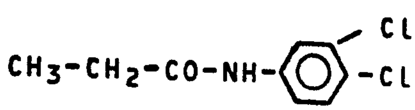

(I-E-5)
(Propanil)

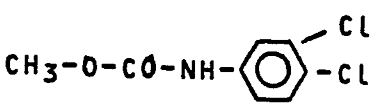

(I-E-6)
(Swep)

(F) Uracil-Derivate der Formeln

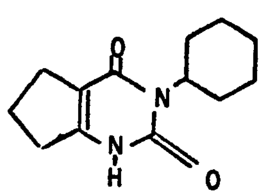

(I-F-1)
(Lenacil)

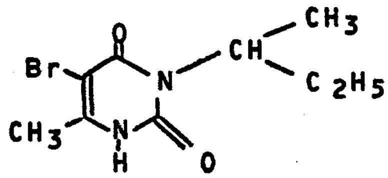

(I-F-2)
(Bromacil)

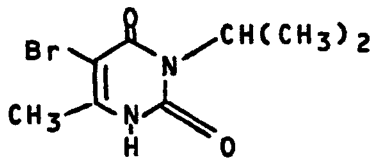

(I-F-3)
(Isocil)

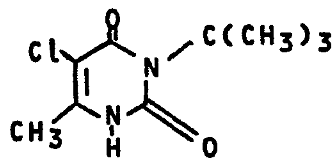

(I-F-4)
(Terbacil)

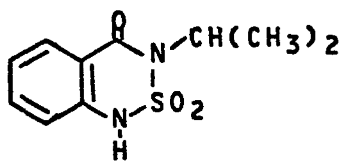

(I-F-5)
(Bentazon)

(G) Biscarbamat-Derivate der Formeln

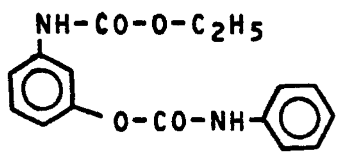

(I-G-1)
(Desmedipham)

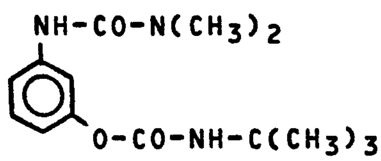

(I-G-2)
(Karbutilate)

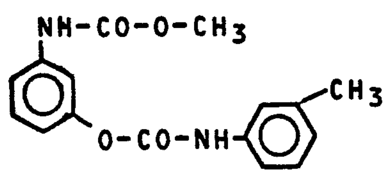

(I-G-3)
(Phenmedipham)

(H) Pyridazinon-Derivate der Formeln

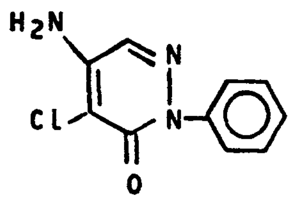

(I-H-1)
(Pyrazone)

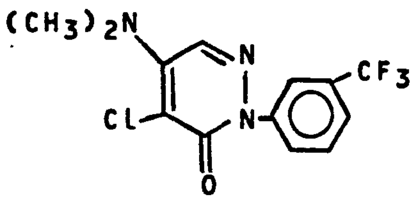

(I-H-2)
(Metflurazone)

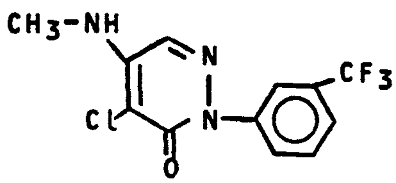

(I-H-3)
(Norflurazon)

(J) Hydroxybenzonitril-Derivate der Formeln

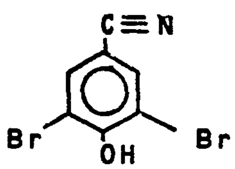

(I -J-1)
(Bromoxynil)

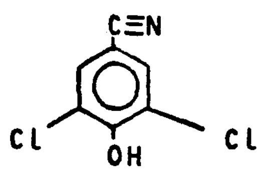

(I -J-2)
(Chloroxynil)

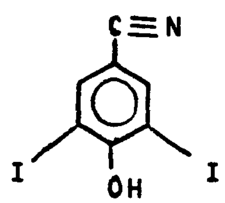

(I -J-3)
(Ioxynil)

Die Photosynthesehemmer-Wirkstoffe der Formeln (I-A)bis (I-J) sind bekannt (vergl. z. B. Carl Fedtke, Biochemistry and Physiology of Herbicide Action, Springer-Verlag, 1982).

Die weiterhin als Mischungskomponente zu verwendenden Pyridincarbonsäureamide sind durch die Formel (II) allgemein definiert. In dieser Formel stehen vorzugsweise

R für geradkettiges oder verzweigtes Alkyl mit 1 bis 6 Kohlenstoffatomen, Fluor, Chlor, Brom, Nitro, Cyano, Alkoxy mit 1 bis 8 Kohlenstoffatomen, sowie für jeweils gegebenenfalls einfach bis dreifach, gleich oder verschieden substituiertes Phenyl und Benzyloxy, wobei als Substituenten im Phenylteil jeweils genannt seien: Halogen, Alkyl mit 1 bis 4 Kohlenstoffatomen, Alkoxy mit 1 bis 2 Kohlenstoffatomen, Nitro und Cyano; und der Index

n für die Zahlen 0, 1, 2 und 3.

Besonders bevorzugt sind Verbindungen der Formel (II), in denen

R für Methyl, Ethyl, Chlor, Nitro, Cyano, Methoxy, sowie für jeweils gegebenenfalls einfach oder zweifach durch Chlor und/oder Methyl substituiertes Phenyl oder Benzyloxy steht, und der Index

n für die Zahlen 0, 1 oder 2 steht.

Die Pyridincarbonsäureamide der Formel (II) sind bekannt (vgl. z.B. Chemica Scripta 13, (1978-79), S. 47; Bulletin of the Chemical Society of Japan 44, (1971), S. 1121-1125; J. Chem. Soc. (C) 1967, S. 1558-1564; US-PS 3 450 706 und DE-OS 26 16 481); bzw. können sie in üblicher Art und Weise erhalten werden, wie z.B. durch Umsetzung der entsprechenden Cyanopyridine mit entsprechenden Alkoholen in Gegenwart einer starken Säure (vgl. auch die Herstellungsbeispiele).

Die Gewichtsverhältnisse der Wirkstoffe in den neuen Wirkstoffkombinationen können in relativ großen Bereichen schwanken. Im allgemeinen entfallen auf ein Gewichtsteil Photosynthesehemmer-Wirkstoff (herbizider Wirkstoff) 0,25 bis 100, vorzugsweise 5 bis 50, insbesondere 10 bis 20, Gew.-Teile Pyridincarbonsäureamid der Formel (II) (Synergist).

Die Photosynthesehemmer-Wirkstoffe weisen starke herbizide Wirkungen auf. Dennoch besitzen sie gegen einige Unkräuter, wie z. B. Galium aparine, Ipomoea hederacea, Datura stramonium, Cirsium arrense, Convolvulus arvensis oder Solanum nigrum und einige Ungräser, wie z. B. Agropyron repens, Avena fatua, Cynodon dactylon, Cyperus ssp. und Colium rigidum eine nicht immer ausreichende Wirkung. Die erfindungsgemäßen Wirkstoffkombinationen dehnen das Wirkungsspektrum der Verbindungen der Formeln (I-A bis I-J) aus und ermöglichen dadurch eine Bekämpfung dieser durch die herbiziden Wirkstoffe alleine nur schwer oder gar nicht bekämpfbaren Unkräuter.

Die erfindungsgemäßen Wirkstoffkombinationen können z.B. bei den folgenden Pflanzen verwendet werden:

Dikotyle Unkräuter der Gattungen: Sinapis, Lepidium, Galium, Stellarium, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffkombinationen ist jedoch keineswegs auf diese Gattungen beschränkt, sondern erstreckt sich in gleicher Weise auch auf andere Pflanzen.

Die erfindungsgemäßen Wirkstoffkombinationen zeigen insbesondere neben einer guten Wirkung gegen grasartige Unkräuter auch eine gute herbizide Wirkung bei breitblättrigen Unkräutern.

Die erfindungsgemäßen Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon,

Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anoranischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

In den Formulierungen können als weitere Zusätze Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90 %.

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch als Einzelformulierungen bei der Anwendung gemischt, d.h. in Form von Tankmischungen zur Anwendung gebracht werden.

Die neuen Wirkstoffkombinationen können als solche oder in ihren Formulierungen weiterhin auch in Mischung mit anderen bekannten Herbiziden Verwendung finden, wobei wiederum Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die erfindungsgemäßen Wirkstoffkombinationen können sowohl vor als auch nach der Einsaat ebenso wie nach dem Auflaufen der Pflanzen gemeinsam oder in getrennten Anwendungen ausgebracht werden. Hierbei spielt die Reihenfolge der Anwendungen keine Rolle.

Beim Einsatz der erfindungsgemäßen Synergisten kann die übliche Aufwandmenge des Herbizids der Formeln (I-A bis I-J) verringert werden. Die Aufwandmenge an herbizidem Photosynthesehemmer-Wirkstoff liegt bei Flächenbehandlung zwischen 0,01 und 3,0 kg/ha, vorzugsweise zwischen 0,05 und 2,0 kg/ha.

Die Aufwandmenge an synergistischem Pyridincarbonsäureamid liegt bei Flächenbehandlung zwischen 0,1 und 10 kg/ha, vorzugsweise zwischen 0,5 und 3 kg/ha.

Die gute herbizide Wirkung der erfindungsgemäßen Wirkstoffkombinationen geht aus den nachfolgenden Beispielen hervor. Während die einzelnen Wirkstoffe in der herbiziden Wirkung Schwächen aufweisen, zeigen die Kombinationen eine Unkrautwirkung, die über eine einfache Wirkungssummierung hinausgeht.

Ein synergistischer Effekt liegt bei Herbiziden immer dann vor, wenn die herbizide Wirkung der Wirkstoffkombination größer ist als die Summe der Wirkungen der applizierten Wirkstoffe.

0138002

Beispiel A

Pre-emergence-Test

Lösungsmittel: 5 Gew.-Teile Aceton
Emulgator: 1 Gew.-Teil Alkylarylpolyglykolether

Zur Herstellung einer zweckmäßigen Wirkstoffzubereitung vermischt man 1 Gew.-Teil herbiziden Wirkstoffs bzw. Synergisten bzw. eines Gemisches aus herbizidem Wirkstoff und Synergisten mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit einer Herbizid-Zubereitung bzw. mit der Synergisten-Zubereitung bzw. mit der Zubereitung aus Synergisten und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Wirkstoffe, Aufwandmenge und Resultate gehen aus den nachfolgenden Tabellen hervor.

Pre-emergence-Test

Tabelle $A_1$

Synergistische Wirkung von Pyridincarbonsäureamiden (II) (= Synergist S) und 4-Amino-6-tert.-butyl-3-methylthio-1,2,4-triazin-5-on (I-A-1) (= Herbizid H) an Ipomoea hederacea. Die Aufwandmenge in kg/ha bezieht sich auf den Gehalt an Wirkstoff.

| Struktur des Synergisten (II) | (S) kg/ha | (H) kg/ha | % Schäden bei Ipomoea hederacea H | S | H+S |
|---|---|---|---|---|---|
| 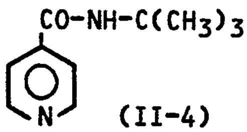 (II-4) | 0,5 | 0,05 | 0 | 0 | 0 |
| | 2 | 0,05 | 0 | 0 | 0 |
| | 0,5 | 0,15 | 0 | 0 | 20 |
| | 2 | 0,15 | 0 | 0 | 20 |
| 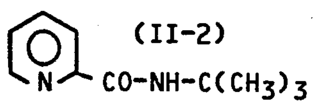 (II-2) | 0,5 | 0,05 | 0 | 0 | 80 |
| | 2 | 0,05 | 0 | 0 | 80 |
| | 0,5 | 0,15 | 20 | 0 | 100 |
| | 2 | 0,15 | 20 | 0 | 100 |
| 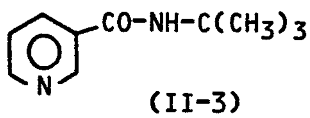 (II-3) | 0,5 | 0,05 | 0 | 0 | 10 |
| | 2 | 0,05 | 0 | 0 | 70 |
| | 0,5 | 0,15 | 40 | 0 | 50 |
| | 2 | 0,15 | 40 | 0 | 100 |
| 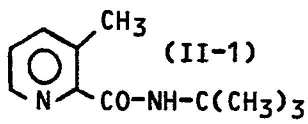 (II-1) | 0,5 | 0,05 | 0 | 0 | 90 |
| | 2 | 0,05 | 0 | 0 | 100 |
| | 0,5 | 0,15 | 30 | 0 | 100 |
| | 2 | 0,15 | 30 | 0 | 100 |
| 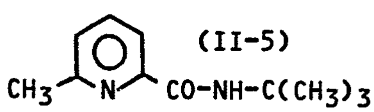 (II-5) | 0,5 | 0,05 | 10 | 0 | 80 |
| | 2 | 0,05 | 10 | 0 | 90 |
| | 0,5 | 0,15 | 20 | 0 | 100 |
| | 2 | 0,15 | 20 | 0 | 100 |
| 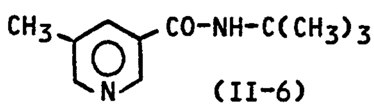 (II-6) | 0,5 | 0,05 | 10 | 0 | 40 |
| | 2 | 0,05 | 10 | 0 | 40 |
| | 0,5 | 0,15 | 20 | 0 | 40 |
| | 2 | 0,15 | 20 | 0 | 100 |

Tabelle $A_1$ (Fortsetzung)

| Struktur des Synergisten | (S) kg/ha | (H) kg/ha | % Schäden bei Ipomoea hederacea H | S | H+S |
|---|---|---|---|---|---|
| 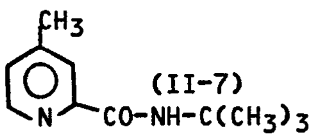 (II-7) | 0,03 | 0,1 | 20 | | 10 |
| | 0,1 | 0,1 | 20 | | 20 |
| | 0,3 | 0,1 | 20 | | 20 |
| | 1 | 0,1 | 20 | | 40 |
| | 2 | 0,1 | 20 | 0 | 100 |
| 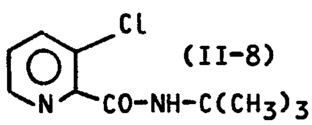 (II-8) | 0,03 | 0,1 | 40 | | 90 |
| | 0,1 | 0,1 | 40 | | 80 |
| | 0,3 | 0,1 | 40 | | 90 |
| | 1 | 0,1 | 40 | | 100 |
| | 2 | 0,1 | 40 | 10 | 100 |
| 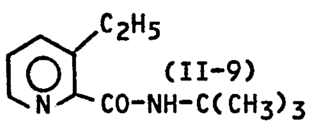 (II-9) | 0,03 | 0,1 | 40 | | 20 |
| | 0,1 | 0,1 | 40 | | 30 |
| | 0,3 | 0,1 | 40 | | 40 |
| | 1 | 0,1 | 40 | | 100 |
| | 2 | 0,1 | 40 | 0 | 100 |
| 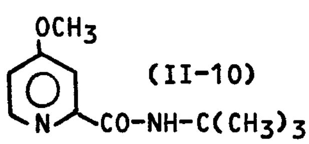 (II-10) | 0,03 | 0,1 | 10 | | 10 |
| | 0,1 | 0,1 | 10 | | 20 |
| | 0,3 | 0,1 | 10 | | 30 |
| | 1 | 0,1 | 10 | | 40 |
| | 2 | 0,1 | 10 | 0 | 60 |
| 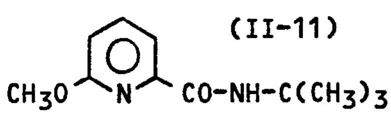 (II-11) | 0,03 | 0,1 | 0 | | 20 |
| | 0,1 | 0,1 | 0 | | 30 |
| | 0,3 | 0,1 | 0 | | 30 |
| | 1 | 0,1 | 0 | | 100 |
| | 2 | 0,1 | 0 | 20 | 100 |

Die alleinige Anwendung des Herbizids (I-A-1) in einer Konzentration von 0,5 bzw. 2,0 kg/ha führt bei Ipomoea hederacea zu einer Schädigung von 80 bzw. 95 %.

## Tabelle $A_2$

Synergistische Wirkung des Pyridincarbonsäureamids gemäß Herstellungsbeispiel (II-2) und verschiedener Photosynthesehemmer-Herbizide an Ipomoea hederacea. Die Aufwandmenge in kg/ha bezieht sich auf den Gehalt an Wirkstoff.

| Synergist | Herst.Bsp. (II-2) | 0 kg/ha | 0,5 kg/ha | 2,0 kg/ha |
|---|---|---|---|---|
| Herbizid | kg/ha | % Wirkung bei Ipomoea hederacea | | |
| Metribuzin (I-A-1) | 0,05 | 30 | 60 | 80 |
| | 0,07 | 30 | 80 | 100 |
| | 0,1 | 40 | 90 | 90 |
| Ametridione (I-B-1) | 0,5 | 50 | 100 | 90 |
| (I-A-2) | 0,3 | 0 | 100 | 60 |
| | 1,0 | 20 | 100 | 100 |
| Methabenzthiazuron (I-D-2) | 3,0 | 40 | 100 | 100 |
| Linuron (I-D-1) | 0,5 | 20 | 50 | 50 |
| | 1,0 | 60 | 80 | 90 |
| Lenacil (I-F-1) | 3,0 | 10 | 40 | 90 |
| Herbizid | 0 | 0 | 0 | 0 |

0138002

Tabelle $A_3$

Synergistische Wirkung des Pyridincarbonsäureamids gemäß Herstellungsbeispiel (II-2) und verschiedener Photosynthesehemmer-Herbizide an Ipomoea hederacea.

Synergist: Herstellungsbeispiel (II-2);
Mischungsverhältnis Herbizid (H): Synergist (S) = 1 : 4

Testpflanze: Ipomoea hederacea

| Behandlung | Herbizid ohne Synergist | | Herbizid + Synergist | |
|---|---|---|---|---|
| | kg/ha H | Wirkung in % | kg/ha H + S | Wirkung in % |
| Metribuzin ± (II-2) (I-A-1) | 0,1 | 3 | 0,1+0,4 | 100 |
| Ametridione ± (II-2) (I-B-1) | 0,2 | 1 | 0,2+0,8 | 63 |
| Atrazine ± (II-2) (I-C-1) | 0,025 | 16 | 0,025+0,1 | 33 |
| Linuron ± (II-2) (I-D-1) | 0,1 | 14 | 0,1+0,4 | 95 |
| --- (II-2) | - | - | 0,0+0,8 | 15 |

Herstellungsbeispiele

Beispiel 1

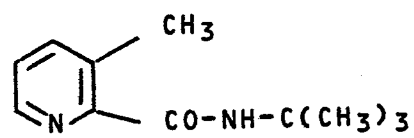

(II-1)

In 100 ml Eisessig werden 111 g (1,5 Mol) tert.-Butanol und 118 g (1 Mol) 3-Methyl-2-cyanopyridin vorgelegt. Dazu wird bei 50°C in 0,5 Stunden 147 g Schwefelsäure (100 %ig) unter Rühren zugetropft und die Mischung 3 Stunden bei 50°C gerührt. Das Oel wird in 1.000 ml Wasser eingerührt und die entstandene Lösung mit konz. Natronlauge unter Eiskühlung auf pH 7 gestellt. Das sich abscheidende Oel wird abgetrennt, in Methylenchlorid aufgenommen und mit Wasser ausgeschüttelt. Die organische Phase wird über Natriumsulfat getrocknent, eingeengt und destilliert. Man erhält 166,5 g (86,7 % der Theorie) 3-Methylpyridin-2-carbonsäure-N-tert.-butylamid als viskoses Oel vom Siedepunkt 155°C/4 mbar.

In analoger Weise können die Verbindungen der allgemeinen Formel (II)

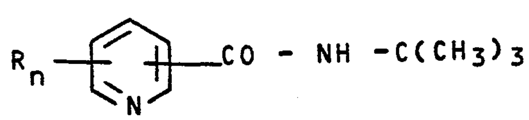

(II)

erhalten werden (s. Tabelle 1):

Tabelle 1 (Fortsetzung)

| Bsp.Nr. | Struktur | Physikalische Konstante |
|---|---|---|
| (II-2) | 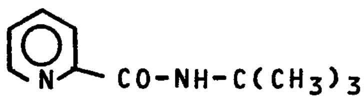 | Kp:175°C/ 20 mbar |
| (II-3) | 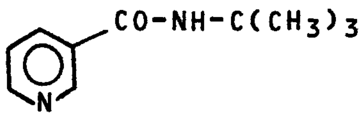 | Fp:79-82°C |
| (II-4) | 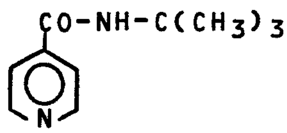 | Fp:119-23°C |
| (II-5) | 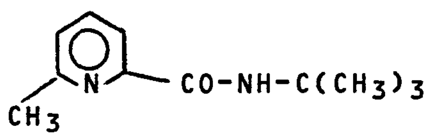 | viskoses Oel |
| (II-6) | 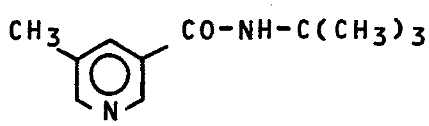 | Fp:107-08°C |
| (II-7) | 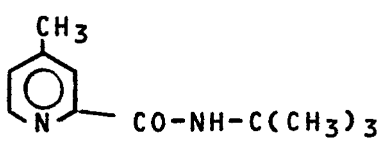 | viskoses Oel |
| (II-8) | 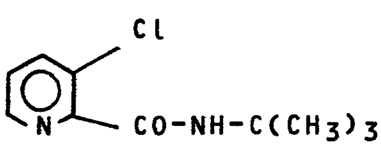 | Fp:103-06°C |
| (II-9) | 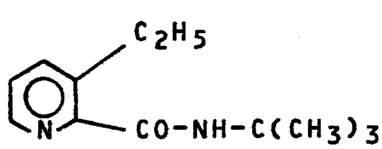 | viskoses Oel |

**Tabelle 1** (Fortsetzung)

| Bsp.Nr. | Struktur | Physikalische Konstante |
|---|---|---|
| (II-10) | 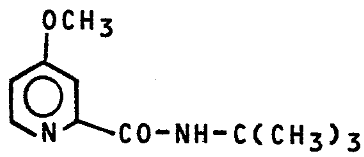 | viskoses Oel |
| (II-11) | 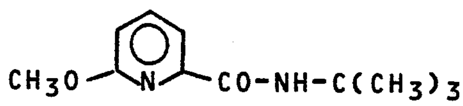 | viskoses Oel |

Patentansprüche

1. Herbizide Mittel, gekennzeichnet durch einen Gehalt an einer Wirkstoffkombination, bestehend aus

(a) einem Photosynthesehemmer-Wirkstoff (Herbizid) und

(b) einem Pyridincarbonsäureamid der allgemeinen Formel (II) (Synergist)

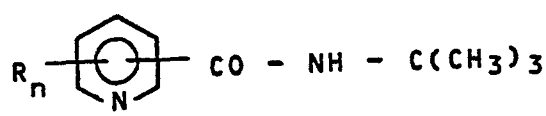
(II)

in welcher

R für Alkyl, Halogen, Nitro, Cyano, Alkoxy, gegebenenfalls substituiertes Phenyl oder gegebenenfalls substituiertes Benzyloxy steht und der Index

n für die Zahlen 0, 1, 2 oder 3 steht.

2. Herbizide Mittel gemäß Anspruch 1, dadurch gekennzeichnet, daß sie als Photosynthesehemmer-Wirkstoffe (Herbizide) Verbindungen aus den nachfolgend genannten Wirkstoffgruppen der Formeln (I-A) bis (I-J) enthalten:

(A) Triazinon-Derivate der Formel

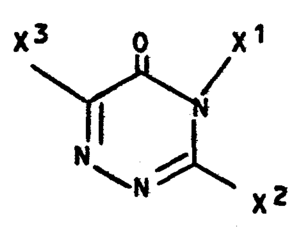
(I-A)

in welcher

$X^1$ für Amino, gegebenenfalls substituiertes Alkylidenamino oder Alkyl mit 1 bis 2 Kohlenstoffatomen steht;

$X^2$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^3$ für gegebenenfalls durch Halogen substituiertes tert.-Butyl oder gegebenenfalls substituiertes Phenyl steht;

(B) Triazindion-Derivate der Formel

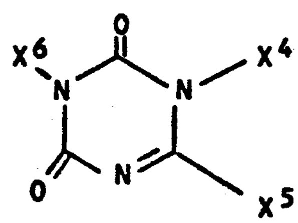

(I-B)

in welcher

$X^4$ für Amino, gegebenenfalls substituiertes Alkylidenamino oder Alkyl mit 1 bis 2 Kohlenstoffatomen steht;

$X^5$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^6$ für Alkyl mit 1 bis 6 Kohlenstoffatomen oder gegebenenfalls substituiertes Phenyl steht;

(C) Triazin-Derivate der Formel

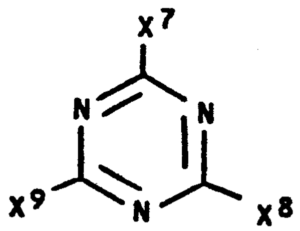

(I-C)

in welcher

$X^7$ für Chlor, Alkoxy oder Alkylthio mit jeweils 1 bis 2 Kohlenstoffatomen steht;

$X^8$ für Alkylamino mit 1 bis 4 Kohlenstoffatomen im Alkylteil steht; und

$X^9$ für gegebenenfalls durch Cyano substituiertes Alkyl mit 1 bis 4 Kohlenstoffatomen steht;

(D) Harnstoff-Derivate der Formel

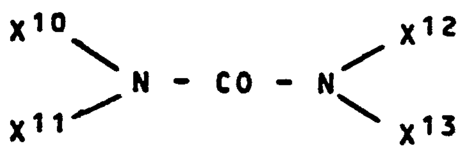

(I-D)

in welcher

$X^{10}$ für gegebenenfalls substituiertes Phenyl, Benz-thiazolyl oder gegebenenfalls substituiertes Thiadiazolyl steht;

$X^{11}$ für Wasserstoff oder Methyl steht;

$X^{12}$ für Methyl steht; und

$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht;

(E) Carboxanilid-Derivate der Formel

$$X^{14} - CO - NH - X^{15} \qquad (I-E)$$

in welcher

$X^{14}$ für Alkyl mit bis zu 6 Kohlenstoffatomen, Alkoxy mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht;

(F) Uracil-Derivate der Formel

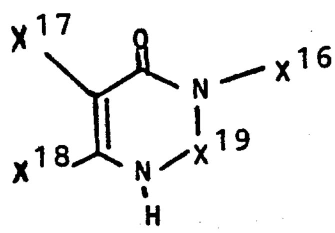

(I-F)

in welcher

$X^{16}$ für Alkyl mit 1 bis 6 Kohlenstoffatomen oder Cycloalkyl mit 5 bis 7 Kohlenstoffatomen steht;

$X^{17}$ für Halogen steht;

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die -CO- oder $-SO_2$-Gruppe steht;

(G) Biscarbamat-Derivate der Formel

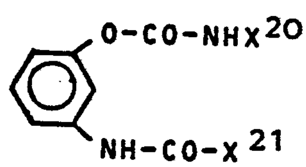

(I-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder Dialkylamino mit 1 bis 2 Kohlenstoffatomen in jedem Alkylteil steht;

(H) Pyridazinon-Derivate der Formel

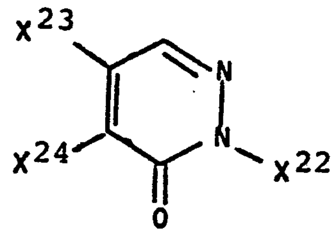

(I-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht;

(J) Hydroxybenzonitril-Derivate der Formel

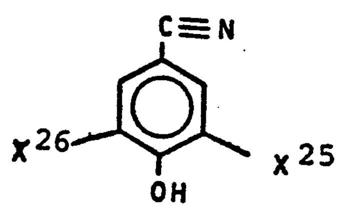

(I-J)

in welcher

$X^{25}$ für Halogen steht; und

$X^{26}$ für Halogen steht.

3. Herbizide Mittel gemäß Anspruch 1, dadurch gekennzeichnet, daß sie als Photosynthesehemmer-Wirkstoff (Herbizid) 4-Amino-6-tert.-butyl-3-methylthio-1,2,4-triazin-5-on der Formel (I-A-1) (Metribuzin)

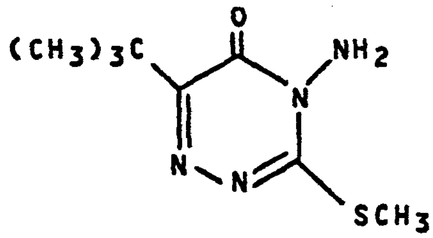

enthalten.

4. Herbizide Mittel gemäß Anspruch 1, dadurch gekennzeichnet, daß in der Wirkstoffkombination das Gewichtsverhältnis von (1) Photosynthesehemmer-Wirkstoff (Herbizid) zu (2) dem Pyridincarbonsäureamid der Formel (II) (Synergist) zwischen 1 : 0,25 und 1 : 100 liegt.

5. Herbizide Mittel gemäß Anspruch 4, dadurch gekennzeichnet, daß das angegebene Gewichtsverhältnis zwischen 1 : 5 und 1 : 50 liegt.

6. Herbizide Mittel gemäß Anspruch 5, dadurch gekennzeichnet, daß das angegebene Gewichtsverhältnis zwischen 1 : 10 und 1 : 20 liegt.

7. Verwendung von Wirkstoffkombinationen gemäß Ansprüchen 1 bis 6 zur Bekämpfung von Unkraut.

8. Verfahren zur Herstellung von herbiziden Mitteln, dadurch gekennzeichnet, daß man eine Wirkstoffkombination gemäß Ansprüchen 1 bis 6 mit Streckmitteln und/oder oberflächenaktiven Mitteln vermischt.

9. Verwendung von Pyridincarbonsäureamiden der Formel (II) gemäß Anspruch 1 als Synergisten in Kombination mit Photosynthesehemmer-Herbiziden.

10. Verwendung von Pyridincarbonsäureamiden der Formel (II) gemäß Anspruch 1 als Synergisten in Kombination mit dem herbiziden Wirkstoff 4-Amino-6-tert.-butyl-3-methylthio-1,2,4-triazin-5-on (Metribuzin) der Formel (I-A-1) gemäß Anspruch 3.